# EUROPEAN PATENT SPECIFICATION

(11) **EP 1 607 097 B1**
(45) Date of publication and mention of the grant of the patent: **26.11.2008**
(21) Application number: 04255051.7
(22) Date of filing: 20.08.2004
(51) Int. Cl.: A61K 36/185, A61K 31/07

(54) **Stabilized compositions containing the oxygen-labile agent retinol**
Stabilisierte Zusammensetzungen, die die sauerstoffempfindliche Verbindung Retinol enthalten
Compositions stabilisées renfermant l'agent retinol qui est labil en présence d'oxygène

(30) Priority: 21.08.2003 US 645915
(43) Date of publication of application: 21.12.2005
(73) Proprietor: NEUTROGENA CORPORATION, Los Angeles California 90045 (US)
(72) Inventor: Marrs, Christopher, Foothill Ranch CA 92610 (US)
(74) Representative: Fisher, Adrian John

(56) References cited:
- WO-A-02/076433
- US-A- 4 146 615
- US-A- 5 455 033
- US-A- 6 039 955
- US-A1- 2002 054 924
- US-A1- 2003 108 627
- US-A1- 2003 152 656
- US-B1- 6 440 465
- US-B1- 6 468 552
- GADOW VON A ET AL: "COMPARISON OF THE ANTIOXIDANT ACTIVITY OF ASPALATHIN WITH THAT OF OTHER PLANT PHENOLS OF ROOIBOS TEA (ASPALATHUS LINEARIS), ALPHA- TOCOPHEROL, BHT, AND BHA" JOURNAL OF AGRICULTURAL AND FOOD CHEMISTRY, AMERICAN CHEMICAL SOCIETY. WASHINGTON, US, vol. 45, no. 3, March 1997 (1997-03), pages 632-638, XP000683366 ISSN: 0021-8561

## Description

### FIELD OF THE INVENTION

The present invention relates to compositions comprising retinol.

### BACKGROUND OF THE INVENTION

It has become desirable to include various oxygen-labile active agents in topical skin care compositions in order to provide a cosmetic/therapeutic benefit, e.g., to the skin and hair. Examples of such active agents include, but are not limited to, vitamins such as vitamin C, vitamin E, vitamin K, and vitamin A. Other active agents such as ubiquinone and hydroquinone can be used to reduce the appearance of aging. Stabilizing compositions containing such oxygen-labile active agents, however, has been proven difficult as such active agents are often either combined with other compounds that may accelerate their decomposition or they are exposed to the environment (e.g., oxygen).

Many commercially available products containing oxygen labile active agents are packaged under nitrogen or other inert gas such as argon and/or in foil-lined tubes and the like. The use of argon and/or foil lined tubes further improves the stability of the oxygen labile active agent, but significantly increases the cost of the product. Therefore, there is a need for an improved composition that stabilizes such oxygen labile active agents (e.g., to improve product performance and/or that eliminates the need for an argon purge and/or foil lined tubes).

### SUMMARY OF THE INVENTION

In one aspect, the present invention features a composition including (i) retinol, and (ii) Chaparral extract. In one embodiment, the present invention features a composition including (i) retinol, (ii) an isoascorbic acid derivative, (iii) a tocopherol derivative, and (iv) Chaparral extract.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed merely to be illustrative and not limitative of the remainder of the disclosure in any way whatsoever.

Unless defined otherwise, all technical and scientific terms used herein have the same meaning as commonly understood by one of ordinary skill in the art to which the invention belongs. As used herein, all isomers are included for compounds (e.g., tocopherol) where no specific isomer is indicated.

The amount of retinol in the composition will depend upon the desired therapeutic/cosmetic effect. In one embodiment the composition comprises from about 0.001% to about 1% (e.g., from about 0.01% to about 0.5%), by weight, of retinol.

In one embodiment, the composition of the present invention includes one or more oil-soluble antioxidants. As used herein, "oil-soluble antioxidant" means an antioxidant which primarily dissolves in the oil phase of an oil-in-water emulsion. Examples of suitable oil-soluble antioxidants include, but are not limited to, tocopherol, ubiquinone, lycopene, astaxanthin, tocotrienol, lutein, polyphenolics, and other carotenoids, and salts and esters thereof.

In one embodiment, the composition contains a tocopherol derivative. What is meant by a "tocopherol derivative" is tocopherol (e.g., α-tocopherol, β-tocopherol, δ-tocopherol, and other unsaturated isomers thereof) and salts or esters thereof (e.g., tocopherol acetate). The amount of oil-soluble antioxidant utilized in the compositions of the present invention may vary, but typically ranges from about 0.1% to about 5%, such as from about 0.25% to about 2% by weight, based on the total weight of the composition.

In one embodiment, the composition further includes one or more water-soluble antioxidants. As used herein, "water-soluble antioxidant" means an antioxidant which primarily dissolves in the aqueous phase of an oil-in-water emulsion. Examples of suitable water-soluble antioxidants include, but are not limited to, sulfites, glutathione, β-glucan, glycosylated polyphenolics, tannins, isoascorbic acid, and ascorbic acid, and salts and esters thereof.

What is meant by an isoascorbic acid derivative is isoascorbic acid and salts and esters thereof. The amount of water-soluble antioxidant utilized in the compositions of the present invention may vary, but typically ranges from about 0.01% to about 1%, such as from about 0.025% to about 0.1% by weight, based on the total weight of the composition.

What is meant by Chaparral extract is the solid extract from the plant. The extract may be solubilized or dispersed in a liquid carrier such as water or organic solvents such as alcohols (e.g., ethanol) or glycols (e.g., butylene glycols). The amount of plant extract utilized in the compositions of the present invention may vary, but typically ranges from about 0.1% to about 10%, such as from about 0.5% to about 5% by weight, based on the total weight of the composition.

The topical compositions useful in the present invention involve formulations suitable for topical application to skin. The compositions may be made into a wide variety of product types that include but are not limited to lotions, creams, gels, sticks, sprays, ointments, shampoos, pastes, mousses, and cosmetics. These product types may comprise several types of cosmetically acceptable carrier systems including, but not limited to solutions, emulsions, gels, solids and liposomes.

What is meant by "cosmetically acceptable carrier" is a carrier that is capable of having the oxygen-labile active agent and the plant extract and/or fungal extract dispersed or dissolved therein, and of possessing acceptable safety properties (e.g., irritation and sensitization characteristics).

The topical compositions useful in compositions of the present invention formulated as solutions typically include an aqueous (e.g., water) or organic solvent (e.g., from about 80% to about 99.99% or from about 90% to about 99% of an acceptable aqueous or organic solvent). Examples of suitable organic solvents include: propylene glycol, polyethylene glycol (200-600), polypropylene glycol (425-2025), glycerol, 1,2,4-butanetriol, sorbitol esters, 1,2,6-hexanetriol, ethanol, and mixtures thereof.

Topical compositions useful in the subject invention may be formulated as a solution comprising one or more emollients. Such compositions typically contain from about 2% to about 50% of a an emollient(s). As used herein, "emollients" refer to materials used for the prevention or relief of dryness, as well as for the protection of the skin.

A lotion can be made from a solution carrier system. Lotions typically comprise from about 1% to about 20% (e.g., from about 5% to about 10%) of an emollient(s) and from about 50% to about 90% (e.g., from about 60% to about 80%) of water.

Another type of product that may be formulated from a solution carrier system is a cream. A cream typically comprises from about 5% to about 50% (e.g., from about 10% to about 20%) of an emollient(s) and from about 45% to about 85% (e.g., from about 50% to about 75%) of water.

Yet another type of product that may be formulated from a solution carrier system is an ointment. An ointment may comprise a simple base of animal or vegetable oils or semi-solid hydrocarbons (oleaginous, absorbent, emulsion and water soluble ointment bases). Ointments may also comprise absorption ointment bases that absorb water to form emulsions. Ointment carriers may also be water-soluble. An ointment may comprise from about 2% to about 10% of an emollient(s) plus from about 0.1% to about 2% of a thickening agent(s).

If the carrier is formulated as an emulsion, typically from about 1% to about 10% (e.g., from about 2% to about 5%) of the carrier system comprises an emulsifier(s). Emulsifiers may be nonionic, anionic or cationic.

Lotions and creams can be formulated as emulsions. Typically such lotions comprise from 0.5% to about 5% of an emulsifier(s). Such creams would typically comprise from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type, are well-known in the cosmetic art and are useful in the subject invention. Multiphase emulsion compositions, such as the water-in-oil-in-water type, are also useful in the subject invention. In general, such single or multiphase emulsions contain water, emollients, and emulsifiers as essential ingredients.

The topical compositions useful in the subject invention may contain, in addition to the aforementioned components, a wide variety of additional oil-soluble materials and/or water-soluble materials conventionally used in topical compositions, at their art-established levels. Various water-soluble materials may also be present in the compositions useful in the subject invention. These include humectants, proteins and polypeptides, preservatives and an alkaline agent. In addition, the topical compositions useful herein can contain conventional cosmetic adjuvants, such as dyes, opacifiers (e.g., titanium dioxide), pigments and perfumes.

The compositions (e.g., the cosmetic compositions) of the present invention can be topically applied to the skin or hair of a mammal (e.g., by the direct laying on or spreading of the composition on the skin or hair of a human). Depending on the selection of the active agent (e.g., the oxygen-labile active agent or other active agents), the compositions can be used to treat a number of skin and hair disorders such as but not limited to acne, mottled hyperpigmentation, age spots, wrinkles, fine lines, cellulite, and other visible signs of aging (whether due to photoaging or chronoaging).

The composition and formulations containing such compositions of the present invention may be prepared using methodology that is well known by an artisan of ordinary skill. The following is a description of the manufacture of various compositions. One of these (Example III) is a composition according to the invention. The others are included by way of comparison. Other compositions of the present invention can be prepared in an analogous manner by a person of ordinary skill in the art.

### Example 1- Manufacture of Emulsion Compositions Containing Retinol

Seven formulations containing retinol (Examples I-VII), as described in Table 1, were manufactured as set forth below.

**Table 1**

| | **Examples** | | | | | | |
|---|---|---|---|---|---|---|---|
| | **I** | **II** | **III** | **IV** | **V** | **VI** | **VII** |
| **Water Phase Ingredients** | **Weight Percentage** | | | | | | |
| Deionized water | 76.40 | 76.40 | 72.34 | 74.43 | 72.34 | 72.35 | 72.34 |
| Carbomer | 0.65 | 0.65 | 0.65 | 0.65 | 0.65 | 0.65 | 0.65 |
| Methyl paraben | 0.35 | 0.35 | 0.35 | 0.35 | 0.35 | 0.35 | 0.35 |
| Disodium edetate | 0.1 | 0.1 | 0.1 | 0.1 | 0.1 | 0.1 | 0.1 |
| D-panthenol | 0.3 | 0.3 | 0.3 | 0.3 | 0.3 | 0.3 | 0.3 |
| Ascorbyl glucoside | 2.1 | 2.1 | 2.1 | 2.1 | 2.1 | 2.1 | 2.1 |
| Acrylates/C10-30 alkyl acrylate crosspolymer | 0.25 | 0.25 | 0.25 | 0.25 | 0.25 | 0.25 | 0.25 |
| Glycerin | 3 | 3 | 3 | 3 | 3 | 3 | 3 |
| Isoascorbic acid | 0 | 0.05 | 0.05 | 0.05 | 0.05 | 0.05 | 0.05 |
| Propyl paraben | 0.17 | 0.17 | 0.17 | 0.17 | 0.17 | 0.17 | 0.17 |
| Phenoxyethanol | 0.73 | 0.73 | 0.73 | 0.73 | 0.73 | 0.73 | 0.73 |

| **Oil Phase Ingredients** | | | | | | | |
|---|---|---|---|---|---|---|---|
| C12-15 alkyl benzoate | 4 | 4 | 4 | 4 | 4 | 4 | 4 |
| ethylhexyl methoxycinnamate | 4 | 4 | 4 | 4 | 4 | 4 | 4 |
| Steareth-10 | 0.5 | 0.5 | 0.5 | 0.5 | 0.5 | 0.5 | 0.5 |
| Butylated hydroxytoluene | 0.1 | 0.1 | 0.1 | 0.1 | 0.1 | 0.1 | 0.1 |
| Dimethicone | 1 | 1 | 1 | 1 | 1 | 1 | 1 |
| Cetyl alcohol | 1 | 1 | 1 | 1 | 1 | 1 | 1 |
| Tocopheryl acetate | 0.5 | 0.5 | 0.5 | 0.5 | 0.5 | 0.5 | 0.5 |
| Octyl hydroxystearate | 1 | 1 | 1 | 1 | 1 | 1 | 1 |

| **Neutralization Ingredient** | | | | | | | |
|---|---|---|---|---|---|---|---|
| Sodium hydroxide (50% solution) | 1.5 | 1.5 | 1.5 | 1.5 | 1.5 | 1.5 | 1.5 |
| Deionized water | 1.1 | 1.1 | 1.1 | 1.1 | 1.1 | 1.1 | 1.1 |

| **Post-addition Ingredients** | | | | | | | |
|---|---|---|---|---|---|---|---|
| Camellia oleifera extract (and) water (and) butylene glycol | 1 | 1 | 1 | 1 | 1 | 0 | 1 |
| Tocopherol | 0.05 | 0.05 | 0.05 | 0.05 | 0.05 | 0.05 | 0.05 |
| Retinol | 0.21 | 0.21 | 0.21 | 0.21 | 0.21 | 0.21 | 0.21 |
| Chaparral extract | 0 | 0 | 4 | 0 | 0 | 0 | 0 |
| Chrysanthellum extract | 0 | 0 | 0 | 2 | 0 | 0 | 0 |
| Olive leaf extract | 0 | 0 | 0 | 0 | 4 | 0 | 0 |
| Lanatellys extract | 0 | 0 | 0 | 0 | 0 | 5 | 0 |
| Lapacho extract | 0 | 0 | 0 | 0 | 0 | 0 | 4 |

The following procedure was used to make each of Examples I-VII. The carbomer (Carbomer Ultrex M, Noveon, Inc. 9911 Brecksville Road Cleveland OH 44141-3247) was added to a primary container followed by the deionized water and allowed to hydrate prior to mixing (5 minutes). The propeller mixer was started and Disodium edetate, Glycerine, D-Panthenol, and Ascorbyl Glucoside, were added and heated to 65-75°C. At 70°C the Phenoxyethanol, Propyl Paraben, Methyl Paraben and Isoascorbic acid were added and mixed until dissolved. The mixture was the homogenized using a Silverson homogenizer, and Acrylates/C10-30 alkyl acrylate crosspolymer ("Pemulen TR-1", Noveon, Inc. 9911 Brecksville Road Cleveland OH 44141-3247)was sprinkled slowly into the mixture at 65% for about 1 minute.

In a second container, the oil phase ingredients, Ethylhexyl methoxy cinnamate, C12-15 alkyl benzoate (Finetex, Elmwood Park, NJ), Octyl hydroxystearate, Dimethicone, Cetyl Alcohol, Butylated hydroxytoluene, Tocopherol Acetate, and Steareth 10 were combined and heated to 65-75°C. The oil phase ingredients were constantly mixed to ensure homogeneity.

After both phases reached the requisite temperature of 65-75°C, the oil phase in the second container was slowly poured and mixed into the water phase in the primary container. After phasing, the mixture was allowed to mix for five minutes. Then the batch was neutralized with the sodium hydroxide to a pH between 6 and 7. The batch was allowed to cool to 45-50°C, and the post-addition ingredients, Tocopherol, Retinol (Retinol 50C, BASF, Mt. Olive, NJ), and Water/Butylene Glycol/Camellia Oleifera Extract (Active Organics, Dallas, TX) followed by the extract of interest as depicted in Table 2.

### Example 2- Manufacture of Emulsion Compositions Containing Retinol With Different Plant Extracts

Five formulations containing retinol (Examples VIII-XII), as described in Table 2, were manufactured as set forth in Example 1.

**Table 2**

| | **Examples** | | | | |
|---|---|---|---|---|---|
| | **VIII** | **IX** | **X** | **XI** | **XII** |
| **Water Phase Ingredients** | **Weight Percentage** | | | | |
| Deionized water | 74.43 | 72.34 | 72.34 | 72.35 | 72.34 |
| Carbomer | 0.65 | 0.65 | 0.65 | 0.65 | 0.65 |
| Methyl paraben | 0.35 | 0.35 | 0.35 | 0.35 | 0.35 |
| Disodium edetate | 0.1 | 0.1 | 0.1 | 0.1 | 0.1 |
| Panthenol | 0.3 | 0.3 | 0.3 | 0.3 | 0.3 |
| Ascorbyl glucoside | 2.1 | 2.10 | 2.1 | 2.1 | 2.1 |
| Acrylates/C10-30 alkyl acrylate crosspolymer | 0.25 | 0.25 | 0.25 | 0.25 | 0.25 |
| Glycerin | 3 | 3 | 3 | 3 | 3 |
| Isoascorbic acid | 0.05 | 0.05 | 0.05 | 0.05 | 0.05 |
| Propyl paraben | 0.17 | 0.17 | 0.17 | 0.17 | 0.17 |
| Phenoxyethanol | 0.73 | 0.73 | 0.73 | 0.73 | 0.73 |

| **Oil Phase Ingredients** | | | | | |
|---|---|---|---|---|---|
| C12-15 alkyl benzoate | 4 | 4 | 4 | 4 | 4 |
| ethylhexyl methoxycinnamate | 4 | 4 | 4 | 4 | 4 |
| Steareth-10 | 0.5 | 0.5 | 0.5 | 0.5 | 0.5 |
| Butylated hydroxytoluene | 0.1 | 0.1 | 0.1 | 0.1 | 0.1 |
| Dimethicone | 1 | 1 | 1 | 1 | 1 |
| Cetyl alcohol | 1 | 1 | 1 | 1 | 1 |
| Tocopheryl acetate | 0.5 | 0.5 | 0.5 | 0.5 | 0.5 |
| Octyl hydroxystearate | 1 | 1 | 1 | 1 | 1 |

| **Neutralization Ingredient** | | | | | |
|---|---|---|---|---|---|
| Sodium hydroxide (50% solution) | 1.5 | 1.5 | 1.5 | 1.5 | 1.5 |
| Deionized water | 1.1 | 1.1 | 1.1 | 1.1 | 1.1 |

| **Post-addition Ingredients** | | | | | |
|---|---|---|---|---|---|
| Camellia oleifera extract (and) water (and) butylene glycol | 1 | 1 | 1 | 1 | 1 |
| Tocopherol | 0.05 | 0.05 | 0.05 | 0.05 | 0.05 |
| Retinol | 0.21 | 0.21 | 0.21 | 0.21 | 0.21 |
| Rooibos extract | 2 | 0 | 0 | 0 | 0 |
| Deionized water | 0 | 2.83 | 2.83 | 2.83 | 2.83 |
| Neem extract | 0 | 1.78 | 0 | 0 | 0 |
| Cranberry extract | 0 | 0 | 1.78 | 0 | 0 |
| Bacopa Monnieri extract | 0 | 0 | 0 | 1.78 | 0 |
| Arjuna extract | 0 | 0 | 0 | 0 | 1.78 |

### Example 3- Manufacture of Emulsion Compositions Containing Retinol With Fungal Extracts or Lactoglobulin

**Four formulations containing retinol (Examples XIII - XVI), as described in Table 3, were manufactured as set forth in Example 1.** The Camellia oleifera extract (and) water (and) trimethylpropane trioctanoate (and) glycerin (and) butylene glycol (and) calcium pantothenate (and) α-tocopherol was purchased from DC Inc. (South Plainsfield, N.J.) under the tradename DC1500 Anti Oxidant Blend®.

**Table 3**

| **Examples** | | | | |
|---|---|---|---|---|
| | **XIII** | **XIV** | **XV** | **XVI** |
| **Water Phase Ingredients** | | | | |
| Deionized water | 43.00 | 71.75 | 72.34 | 71.51 |
| Carbomer | 0.65 | 0.65 | 0.65 | 0.65 |
| Methyl paraben | 0.35 | 0.35 | 0.35 | 0.35 |
| Ergothioneine | 33.33 | 0 | 0 | 0 |
| Propyl paraben | 0.17 | 0.17 | 0.17 | 0.17 |
| Disodium edetate | 0.1 | 0.1 | 0.1 | 0.1 |
| Panthenol | 0.3 | 0.3 | 0.3 | 0.3 |
| Ascorbyl glucoside | 2.1 | 2.1 | 2.1 | 2.1 |
| Acrylates/C10-30 alkyl acrylate crosspolymer | 0.25 | 0.25 | 0.25 | 0.25 |
| Glycerin | 3 | 3 | 3 | 3 |
| Isoascorbic acid | 0.05 | 0.05 | 0.05 | 0.05 |
| Phenoxyethanol | 0.73 | 0.73 | 0.73 | 0.73 |

| **Oil Phase Ingredients** | | | | |
|---|---|---|---|---|
| C12-15 alkyl benzoate | 4 | 4 | 4 | 4 |
| ethylhexyl methoxycinnamate | 4 | 4 | 4 | 4 |
| Steareth-10 | 0.5 | 0.5 | 0.5 | 0.5 |
| Butylated hydroxytoluene | 0.1 | 0.1 | 0.1 | 0.1 |
| dimethicone | 1 | 1 | 1 | 1 |
| Cetyl alcohol | 1 | 1 | 1 | 1 |
| Tocopheryl acetate | 0.5 | 0.5 | 0.5 | 0.5 |
| Octyl hydroxystearate | 1 | 1 | 1 | 1 |

| **Neutralization Ingredient** | | | | |
|---|---|---|---|---|
| Sodium hydroxide (50% solution) | 1.5 | 1.5 | 1.5 | 1.5 |
| Deionized water | 1.1 | 1.1 | 1.1 | 1.1 |

| **Post-addition Ingredients** | | | | |
|---|---|---|---|---|
| Camellia oleifera extract (and) water (and) butylenes glycol | 1 | 1 | 1 | 1 |
| Tocopherol | 0.05 | 0.05 | 0.05 | 0.05 |
| Retinol | 0.21 | 0.21 | 0.21 | 0.21 |
| Deionized water | 0 | 2.83 | 0 | 0 |
| *Phellinus Linteus* extract | 0 | 1.78 | 0 | 0 |
| Camellia oleifera extract (and) water (and) trimethylpropane trioctanoate (and) glycerin (and) butylene glycol (and) calcium pantothenate (and) alpha-tocopherol | 0 | 0 | 4 | 4 |
| Lactoglobulin | 0 | 0 | 0 | 0.83 |

### Example 4- Chemical Stability of Retinol

A study was conducted to determine the impact of the plant extracts on the stability of the oxygen labile active agent Retinol.

Example I-XVI were prepared and packaged in aluminum tubes that were not purged with argon. The formulations were then exposed to different storage conditions. The formulations were set up at 50°C. Samples were taken at 1 month and three months of storage, and analyzed for Retinol content. Table 4 shows the result of the analysis.

**Table 4**

| % Retinol Lost | | |
|---|---|---|
| Example | 1 Month 50°C | 3 Months 50°C |
| I | 32 | 38 |
| II | 7 | 21 |
| III | 1 | 3 |
| IV | 1 | 5 |
| V | 2 | 5 |
| VI | 4 | 12 |
| VII | 7 | 11 |
| VIII | 3 | 11 |
| IX | 0 | 10 |
| X | 4 | 9 |
| XI | 5 | 19 |
| XII | 9 | 22 |
| XIII | 4 | 7 |
| XIV | 4 | 12 |
| XV | 6 | 29 |
| XVI | 5 | 8 |

Example I performed poorly, as after 3 months at 50°C, 38% of the initial concentration of retinol was lost. Under the same conditions, Example II, which further contained isoascorbic acid, performed slightly better, losing 21% of the retinol. However, when Chaparral extract (Active Organics), was further added (Example III), the retention of the retinol was unexpectedly increased.

It is understood that while the invention has been described in conjunction with the detailed description thereof, that the foregoing description is intended to illustrate, and not limit the scope of the invention, which is defined by the scope of the appended claims. Other aspects, advantages, and modifications are within the claims.

## Claims

1. A composition comprising:
(a) retinol, and
(b) Chaparral extract.

2. A composition of claim 1, wherein said composition comprises (i) from .01% to 1%, by weight, of said retinol and (ii) from 0.1% to 10%, by weight, of said chaparral extract.

3. A composition of claim 1, further comprising:
(c) an isoascorbic acid derivative,
(d) a tocopherol derivative.

4. A composition of claim 3, wherein said composition comprises:
(a) 0.001 to 20%, by weight, of retinol,
(b) 0.001% to 0.5%, by weight, of said isoascorbic acid derivative, and
(c) 0.1% to 1%, by weight, of said tocopherol derivative; and
(d) 0.1% to 10%, by weight, of said Chaparral extract.

## Patentansprüche

1. Zusammensetzung, umfassend:
(a) Retinol und
(b) Chaparral-Extrakt.

2. Zusammensetzung nach Anspruch 1, wobei die Zusammensetzung (i) von 0,01 bis 1 Gew.-% vom Retinol und (ii) von 0,1 bis 10 Gew.-% vom Chaparral-Extrakt umfasst.

3. Zusammensetzung nach Anspruch 1, weiter umfassend:
(c) ein Isoascorbinsäure-Derivat,
(d) ein Tocopherol-Derivat.

4. Zusammensetzung nach Anspruch 3, wobei die Zusammensetzung:
(a) 0,001 bis 20 Gew.-% Retinol,
(b) 0,001 bis 0,5 Gew.-% vom Isoascorbinsäure-Derivat und
(c) 0,1 bis 1 Gew.-% vom Tocopherol-Derivat; und
(d) 0,1 bis 10 Gew.-% vom Chaparral-Extrakt
umfasst.

## Revendications

1. Composition comprenant :
(a) du rétinol, et
(b) un extrait de Chaparral.

2. Composition selon la revendication 1, dans laquelle ladite composition comprend (i) de 0,01 à 1 % en poids dudit rétinol et (ii) de 0,1 % à 10 % en poids dudit extrait de Chaparral.

3. Composition selon la revendication 1, comprenant en outre :
(c) un dérivé d'acide iso-ascorbique
(d) un dérivé de tocophérol.

4. Composition selon la revendication 3, dans laquelle ladite composition comprend :
(a) 0,001 à 20 % en poids de rétinol,
(b) 0,001 % à 0,5 % en poids dudit dérivé d'acide iso-ascorbique ; et
(c) 0,1 % à 1 % en poids dudit dérivé de tocophérol ; et
(d) 0,1 % à 10 % en poids dudit extrait de Chaparral.
